# EUROPÄISCHE PATENTSCHRIFT

(11) **EP 1 220 787 B1**
(45) Veröffentlichungstag und Bekanntmachung des Hinweises auf die Patenterteilung: **17.09.2003**
(21) Anmeldenummer: 00962281.2
(22) Anmeldetag: 27.07.2000
(51) Int. Cl.: B65B 55/10, B65B 55/02, B67C 7/00

(54) **VERFAHREN ZUR STERILISATION VON PET-FLASCHEN**
METHOD FOR STERILISING PET BOTTLES
PROCEDE DE STERILISATION DE BOUTEILLES EN PET

(30) Priorität: 15.10.1999 DE 19949692
(43) Veröffentlichungstag der Anmeldung: 10.07.2002
(73) Patentinhaber: GEA Finnah GmbH, 48683 Ahaus (DE)
(72) Erfinder: NIEHR, Thomas, 48683 Ahaus (DE); STEINHAUSER, Ulrich, 35396 Giessen (DE); WEGNER, Herbert, 48683 Ahaus (DE)
(74) Vertreter: Busse & Busse Patentanwälte
(86) Internationale Anmeldenummer: EP0007214
(87) Internationale Veröffentlichungsnummer: WO01028863

(56) Entgegenhaltungen:
- EP-A- 0 375 166
- EP-A- 0 427 051
- EP-A- 0 481 361
- WO-A-79/01074
- DE-A- 3 339 930
- FR-A- 2 666 299
- US-A- 4 797 255

## Beschreibung

Die Erfindung betrifft ein Verfahren zur Sterilisation von entlang einem Förderweg taktweise vorbewegten Behältnissen durch Einsatz eines Steritisationsmittels, bei dem in den Innenraum des Behältnisses erwärmtes Peroxid-Aerosol eingeblasen und auf der Innenwand der Behältnisse ein Peroxid-Kondensatfilm gebildet wird, und bei dem anschließend erwärmte Luft in den Innenraum der Behältnisse eingeblasen wird, bis das Aerosol-Kondensat verdampft ist.

Bei einem bekannten Verfahren dieser Art (US-A-4 797 255) erfolgt der Sterilisationsvorgang in zwei Stufen. Zunächst wird Peroxid in Gasform mit einer Temperatur von 140°C bis 180°C zugeführt, das unmittelbar bei der Kondensatbildung den Sterilisationsvorgang übernimmt, während das nachfolgende Einführungen von erwärmter Luft lediglich dem Trocknen dient.

Bei einem ferner bekannten Verfahren der eingangs genannten Art (EP-A-0 481 361) wird Peroxid mit 108°C, d.h. mit voller Aktivierungstemperatur, zur Durchführung eines einstufigen Kondensations- und Sterilisationsvorganges zugeführt und anschließend Luft mit 250°C zum Trocknen in einer Trocknungsstufe eingesetzt. Beide mit Umgebungsluft arbeitenden Verfahren sind ungeeignet, bei temperaturempfindlichen Flaschen eingesetzt zu werden, da bei den vorgesehenen Temperaturverhältnissen die für derartige Flaschen kritische Temperaturgrenze überschritten werden würde.

Die Erfindung befaßt sich mit dem Problem, ein Verfahren der genannten Art zu schaffen, das eine Sterilisation auch von temperaturempfindlichen Flaschen mit Hilfe von Peroxid als Sterilisationsmittel erlaubt und dabei einfach und schnell innerhalb einer kurzen Förderstrecke durchführbar ist.

Das Verfahren nach der Erfindung löst das Problem mit den Merkmalen des Anspruchs 1. Hinsichtlich weiterer Ausgestaltungen wird auf die Ansprüche 2 bis 15 verwiesen.

Das erfindungsgemäße Verfahren ermöglicht eine Sterilisation der Flaschen mit Hilfe eines Peroxid-Nebels, der zur kurzzeitigen Bildung eines Kondensats auf der Innenfläche der Flaschen in diese eingeblasen wird, wobei sich das Peroxid infolge seiner Erwärmung auf eine Sterilisations-Starttemperatur bereits in einem Stadium befindet, bei dem schon beim Einbringen ein gewisser Teil des Peroxids in Gasform übergeht. Durch das Einblasen von Sterilluft mit einer Aktivierungstemperatur für das Peroxid wird das Peroxid unter gleichzeitigem Auflösen des Kondensatfilms durch Sauerstoffabspaltung aktiviert, führt die Sterilisation herbei und wird anschließend mit verbliebenen Restbestandteilen aus dem Innenraum der Flaschen ausgeblasen. Bei diesen Vorgängen wird eine Wandtemperatur von 55 °C nicht überschritten, so daß das empfindliche Material der Flaschen, wie es insbesondere bei PET-Flaschen gegeben ist, keine Beeinträchtigungen erfährt.

Weitere Einzelheiten und Wirkungen des Gegenstands der Erfindung ergeben sich aus der nachfolgenden Beschreibung des Verfahrensablaufes anhand einer Skizze, die den taktweisen Durchlauf von Flaschengruppen durch eine Sterilisationsstation erfindungsgemäßer Art veranschaulicht.

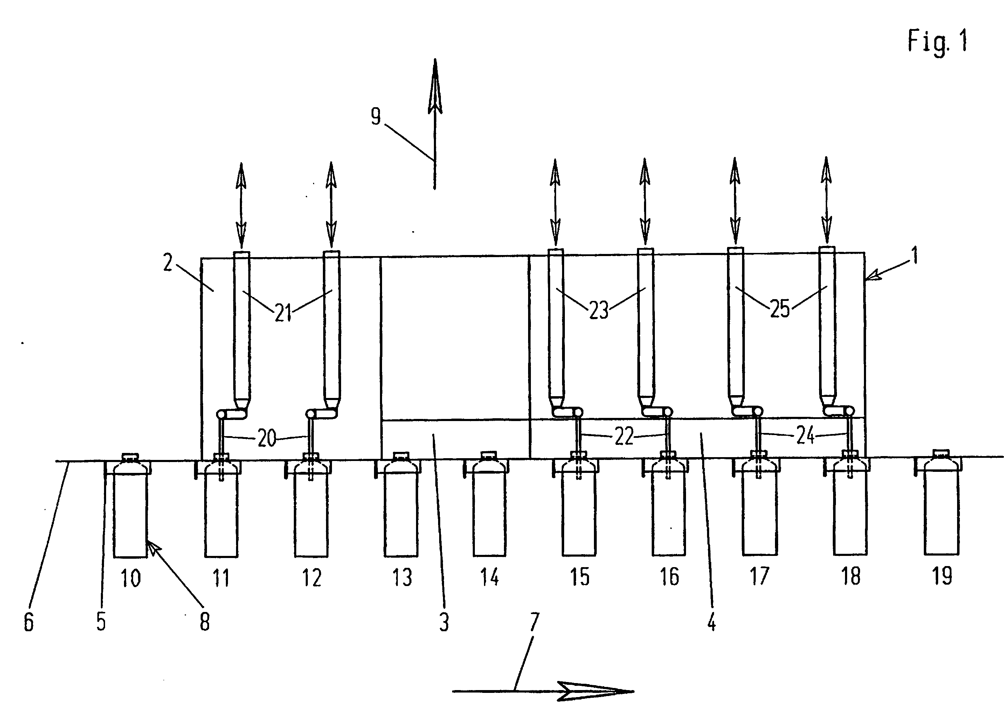

Im einzelnen zeigt die Zeichnung schematisch eine Sterilkammer 1, die einen Einsprühraum 2, einen ersten Steritraum 3 und einen zweiten Sterilraum 4 ausbildet, die jeweils von Gruppen von z.B. zehn in der Zeichenebene fluchtend hintereinander gelegenen Flaschen 8 durchlaufen werden. Dabei stützen sich die Flaschen 8 jeweils in Flaschenträgern 5 in Balkenform ab, die mittels eines nur schematisch durch eine strichpunktierte Linie wiedergegebenen Förderers 6 horizontal in Richtung des Pfeiles 7 gefördert werden. Die Förderung erfolgt schrittweise im Takt, wodurch die Flaschengruppen jeweils die mit 10 bis 19 bezeichneten Stillstandspositionen durchlaufen. Es versteht sich, daß der Position 10 weitere Positionen vorgelagert sein und der Position 19 weitere Positionen nachfolgen können, in denen jeweils auf die Flaschen eingewirkt wird, beispielsweise durch vorausgehende Spül- und Trocknungsvorgänge und durch nachfolgende Befüll- und Verschließvorgänge.

In der Position 11 wird in die Flaschen 8 aus temperaturempfindlichem Kunststoff, insbesondere PET-Flaschen, ein auf eine Sterilisations-Starttemperatur erwärmtes Peroxid-Aerosol eingeblasen, was mit Hilfe einer Lanze 20 erfolgt, die mitsamt ihrer Zuleitung 21 in Richtung des Pfeiles 9 aus einer oberen Ausgangsstellung in die dargestellte untere Betriebsstellung mit Hilfe eines nicht näher veranschaulichten Antriebs bewegbar ist. Die Zuleitung 21 führt zu einem nicht dargestellten Peroxid-Aerosol-Erzeuger, der Peroxid-Aerosol mit Überdruck erzeugt und bei abgesenkter Lanze 20 ein Einblasen des Peroxid- bzw. H₂O₂-Nebels in den Innenraum der Flaschen 8 bewirkt. Der in die Flaschen 8 über die Lanzen 20 eingebrachte Peroxid-Nebel hat eine Sterilisations-Starttemperatur von etwa 60 bis 90 °C und vorzugsweise von etwa 70 bis 80 °C, bei der schon ein gewisser Teil des H₂O₂ unter Abspaltung von Sauerstoff in Gasform übergeht. Dennoch ist die Temperatur so niedrig, daß auch bei Wiederholung des gleichen Vorganges in der Position 12 bei der Bildung eines Kondensatfilmes auf der Innenseite der Flaschen 8 deren Flaschenwandung keine Erwärmung erfährt, die in eine bedrohliche Nähe zur Grenztemperatur von beispielsweise 55 °C kommt.

Nach diesem zweistufigen Einbringen von Peroxid-Aerosol in die Flaschen 8 gelangen diese aus dem Einsprühraum 2 der Sterilkammer 1 in die erste Sterilkammer 3, die wie die Sterilkammer 4 niedriger ausgeführt ist. in dem ersten Sterilraum 3 verweilen die mit Aerosol innenseitig eingesprühten Flaschen 8 in den beiden Positionen 13 und 14 ohne weitere Einwirkung von außen, wonach sie dann in die Position 15 übergehen, wo sie durch eine Lanze 22, die mitsamt ihrer Zuleitung 23 in Richtung des Pfeiles 9 ebenfalls aus einer oberen Ausgangsstellung in die dargestellte Betriebsstellung absenkbar ist und umgekehrt, mit Sterilluft beaufschlagt werden.

Diese in den Positionen 15 und 16 eingeblasene Sterilluft hat eine Aktivierungstemperatur von etwa 90 bis 120 °C, vorzugsweise etwa 110 °C, und bewirkt durch ihr Einblasen, daß der Kondensatfilm auf der Innenfläche der Flaschen verdampft wird. Dieser Vorgang des Auflösens des Aerosol-Kondensatfilms ist auf zwei Stufen in den Positionen 15 und 16 verteilt und erst in der Position 16 abgeschlossen, in der sich der gleiche Einblasvorgang von auf Aktivierungstemperatur erwärmter Sterilluft wiederholt. Das Einblasen der Sterilluft mit Aktivierungstemperatur erfolgt nur kurzzeitig in einem Zeitraum von etwa 1 bis 3 Sekunden, vorzugsweise 2 Sekunden, und dabei mit einer Strömungsgeschwindigkeit von etwa 25 bis 30 m/s, vorzugsweise etwa 28 m/s. Trotz des Wärmeinhalts der auf Aktivierungstemperatur erwärmten Sterilluft verbleibt die Wandung der Flaschen 8 auch in den Positionen 15 und 16 in einem Temperaturbereich unterhalb der Grenztemperatur von etwa 55 °C.

Zum Austreiben von Peroxid-Resten aus dem Innenraum der Flaschen 8 und zuverlässigen Trocknen der Flaschen an ihrer Innenwand wird in den Positionen 17 und 18 jeweils erneut Sterilluft eingeblasen, jedoch mit verminderter Temperatur, um auch in diesen beiden Positionen zu vermeiden, daß die Grenztemperatur in der Flaschenwandung erreicht wird. Diese geminderte Temperatur der Sterilluft in den Positionen 17 und 18 beträgt etwa 75 bis 85 °C, vorzugsweise etwa 80 °C, und wird mit einer Strömungsgeschwindigkeit von etwa 70 bis 90 m/s, vorzugsweise etwa 80 m/s, in den Innenraum der Flaschen eingeblasen, und zwar ebenfalls wieder über einen Zeitraum von nur etwa 1 bis 3 Sekunden, vorzugsweise ebenfalls etwa 2 Sekunden. Die Sterilluft verminderter Temperatur bewirkt zwar ein wirksames Austreiben von Peroxid-Resten und sicheres Abtrocknen der Flaschen 8 an ihrer Innenseite, vermeidet jedoch ebenfalls einen Übergang von Wärme an die Flaschen 8 in einem Ausmaß, das eine Aufwärmung der Flaschenwandungen über Grenztemperatur herbeiführen könnte. Das Einblasen geschieht (ähnlich wie in den Positionen 11 und 12 bzw. 15 und 16) jeweils mit Hilfe einer Lanze 24, die mit einer Zuleitung 25 verbunden und samt dieser in Richtung des Pfeiles 9 aus einer oberen Ausgangsstellung abwärts in die dargestellte untere Betriebsstellung und umgekehrt bewegbar ist. Die Lanzen 20 in den Positionen 11 und 12, 22 in den Positionen 15 und 16 und 24 in den Positionen 17 und 18 können jeweils mittels ein und desselben Antriebs jeweils gemeinsam auf- und abbewegbar sein.

Mit Abschluß des Einblasens von Sterilluft in die Flaschen 8 in Position 18 ist der Sterilisationsvorgang abgeschlossen. Nach Verlassen der Position 18 und des Sterilraumes 4 gehen die Flaschen in Position 10 und folgenden Positionen in beispielsweise einen weiteren Sterilraum über, in dem eine Flaschenbefüllung und ein Verschließen befüllter Flaschen stattfindet.

Die Menge des eingesetzten Aerosols, das vorzugsweise bei Umgebungstemperatur vernebelt und erst auf dem Wege zu den Lanzen 21 auf die Sterilisations-Starttemperatur erwärmt wird, hängt von der Flaschengröße ab und beträgt vorzugsweise etwa 0,15 ml Peroxid je 100 cm² Innenraumfläche der Flaschen 8. Grundsätzlich ist es möglich, das Einblasen von Peroxid-Aerosol in einer einzigen Stufe, z.B. in der Position 11, durchzuführen und die Bearbeitungspause auf eine Stufe, z.B. in Position 13, zu verkürzen. Auch die Einblasvorgänge können jeweils in einer einzigen Stufe, z.B. in Position 15 und 17 erfolgen. Die Aufteilung auf jeweils mehrere Stufen ist jedoch hinsichtlich des Verlaufs des Wärmeüberganges zu den Flaschenwänden hin günstiger im Sinne eines Vermeidens eines Temperaturanstiegs auf die Grenztemperatur. Grundsätzlich können anstelle von zwei Stufen auch jeweils mehr als zwei Stufen für das Einblasen von Peroxid-Aerosol und für die Bearbeitungspause und für das Einblasen von Sterilluft vorgesehen sein, jedoch geht damit eine Verlängerung der Sterilisationszeit und der -strecke einher, die entsprechend erhöhte Kosten verursachen.

Nicht dargestellt aber vorhanden sind die Wandbereiche der Sterilkammer 1 unterhalb und seitlich der Flaschengruppen 8. Nicht dargestellt aber vorhanden sind auch entsprechende Schleusen an dem Übergang der Flaschengruppen aus der Position 10 in die Position 11 und aus der Position 18 in die Position 19.

## Patentansprüche

1. Verfahren zur Sterilisation von entlang einem Förderweg tatktweise vorbewegten Behältnissen durch Einsatz eines Sterilisationsmittels, bei dem in den Innenraum der Behältnisse erwärmtes Peroxyd-Aerosol eingeblasen und auf der Innenwand der Behältnisse ein Peroxyd-Kondensatfilm gebildet wird, und bei dem anschließend erwärmte Luft in den Innenraum der Behältnisse eingeblasen wird, bis das Aerosol-Kondensat verdampft ist, **dadurch gekennzeichnet, daß** zur Sterilisation von Flaschen aus temperaturempfindlichem Kunststoff, insbesondere PET-Flaschen, zur Kondensatfilmbildung Peroxyd-Aerosol in die Flaschen lediglich mit einer Sterilisations-Starttemperatur eingeblasen wird, anschließend bis zum Auflösen des Kondensatfilms durch Verdampfen Sterilluft mit einer die Sterilisations-Starttemperatur des Peroxyd-Aerosols übersteigenden Aktivierungstemperatur eingeblasen wird, und danach durch in einem weiteren gesonderten Schritt erfolgendes Einblasen von Sterilluft Peroxydreste aus dem Innenraum der Flaschen ausgetrieben werden.

2. Verfahren nach Anspruch 1, **dadurch gekennzeichnet, daß** das Peroxid bei Umgebungstemperatur zum Aerosol vernebelt und auf dem Wege zum Innenraum der Flaschen auf Sterilisations-Starttemperatur erwärmt wird.

3. Verfahren nach Anspruch 1 oder 2, **dadurch gekennzeichnet, daß** das Peroxid-Aerosol vor dem Einbringen in die Flaschen auf etwa 60 bis 90 °C, vorzugsweise etwa 70 bis 80 °C, als Sterilisations-Starttemperatur erwärmt wird.

4. Verfahren nach einem der Ansprüche 1 bis 3, **dadurch gekennzeichnet, daß** das Peroxid-Aerosol vor seinem Einblasen in die Flaschen in einen auf Aktivierungstemperatur erwärmten Sterilluftstrom eingebracht und durch diesen auf dem Wege zum Innenraum der Flaschen auf die Sterilisations-Starttemperatur erwärmt wird.

5. Verfahren nach einem der Ansprüche 1 bis 3, **dadurch gekennzeichnet, daß** das Peroxid-Aerosol und die Sterilluft bis zum Eintritt in den Innenraum der Flaschen getrennt geführt werden.

6. Verfahren nach einem der Ansprüche 1 bis 5, **dadurch gekennzeichnet, daß** das Einbringen von Peroxid-Aerosol in den Innenraum der Flaschen in zumindest zwei getrennten, aufeinanderfolgenden Schritten vorgenommen wird.

7. Verfahren nach einem der Ansprüche 1 bis 6, **dadurch gekennzeichnet, daß** dem Einblasen von auf Aktivierungstemperatur erwärmter Sterilluft in den Innenraum der Flaschen zumindest eine dem Einblasen von Peroxid-Aerosol nachfolgende, zumindest einen Fördertakt der Flaschen entsprechende Einwirkungspause vorgeschaltet wird.

8. Verfahren nach einem der Ansprüche 1 bis 7, **dadurch gekennzeichnet, daß** das nach der Bildung des Peroxyd-Kondensatfilms erfolgende Einblasen von Sterilluft in zumindest zwei getrennten, jeweils einen Fördertakt der Flaschen entsprechenden Schritten vorgenommen wird.

9. Verfahren nach einem der Ansprüche 1 bis 8, **dadurch gekennzeichnet, daß** die Sterilluft auf eine Aktivierungstemperatur von etwa 90 bis 120 °C, vorzugsweise etwa 110 °C, erwärmt wird.

10. Verfahren nach einem der Ansprüche 1 bis 9, **dadurch gekennzeichnet daß** die nach dem Einblasen von Sterilluft mit Aktivierungstemperatur eingeblasene Sterilluft eine geminderte Temperatur aufweist.

11. Verfahren nach Anspruch 10, **dadurch gekennzeichnet, daß** die geminderte Temperatur der Sterilluft etwa 75 bis 85 °C, vorzugsweise etwa 80°C, beträgt.

12. Verfahren nach einem der Ansprüche 1 bis 11, **dadurch gekennzeichnet, daß** die Sterilluft mit Aktivierungstemperatur mit einer Strömungsgeschwindigkeit von etwa 25 bis 30 m/s, vorzugsweise etwa 28 m/s, in den Innenraum der Flaschen eingeblasen wird.

13. Verfahren nach einem der Ansprüche 1 bis 12, **dadurch gekennzeichnet, daß** die Sterilluft mit geminderter Temperatur mit einer Strömungsgeschwindigkeit von etwa 70 bis 90 m/s, vorzugsweise etwa 80 m/s, in den Innenraum der Flaschen eingeblasen wird.

14. Verfahren nach einem der Ansprüche 1 bis 12, **dadurch gekennzeichnet, daß** das Einblasen von Sterilluft jeweils über einen Zeitraum von etwa 1 bis 3 Sekunden, vorzugsweise etwa 2 Sekunden, vorgenommen wird.

15. Verfahren nach einem oder mehreren der Ansprüche 1 bis 14, **dadurch gekennzeichnet, daß** etwa 0,15 ml Peroxid je 100 cm² Innenraumfläche der Flaschen in diese eingebracht wird.

## Claims

1. Procedure for the sterilisation of containers moving at intervals along a conveyor path by means of sterilising agent such that heated peroxide aerosol is blown into the interior space of the containers and then forms a film of peroxide condensate on the inner wall of the containers and that, subsequently, heated air is blown into the interior space of the containers until the aerosol condensate has evaporated, **characterised in that** to achieve sterilisation of bottles made of temperature-sensitive plastic material, in particular bottles made of PET, peroxide aerosol is blown into the bottles at only sterilisation-initiating temperature in order to form a condensate film after which, in order to disperse the condensate film by evaporation, sterile air at an activating temperature higher than that of the sterilisation-initiating temperature for the peroxide-aerosol is blown in and, subsequently in a further separate step sterile air is blown in to drive peroxide residues out of the interior space of the bottles.

2. Procedure in accordance with claim 1, **characterised in that** the peroxide atomises at the ambient temperature to form an aerosol which is then heated to the sterilisation-initiating temperature during its passage to the inner space of the bottle.

3. Procedure in accordance with claim 1 or 2, **characterised in that** before passing into the bottles the peroxide aerosol is heated to a sterilisation-initiating temperature of between 60 and 90°C and, preferably, to between 70 and 80 °C.

4. Procedure in accordance with one of the claims 1 to 3, **characterised in that** before being blown into the bottles the peroxide-aerosol is mixed with a stream of sterile air heated to activation temperature and is heated by this air stream to the sterilisation-initiating temperature as it passes to the inner space of the bottles.

5. Procedure in accordance with one of the claims 1 to 3, **characterised in that** the peroxide aerosol and the sterile air follow separate supply channels up to the point of entry into the inner space of the bottles.

6. Procedure in accordance with one of the claims 1 to 5, **characterised in that** the introduction of the peroxide aerosol into the inner space of the bottles takes place in at least two separate and successive steps.

7. Procedure in accordance with one of claims 1 to 6, **characterised in that** at least one pause for the desired effect to be achieved precedes the blowing-in of sterile air heated to activation temperature into the inner space of the bottles in such a manner that the pause not only follows the blowing-in of peroxide but also coincides with at least one phased step in the transport of the bottles.

8. Procedure in accordance with one of the claims 1 to 7, **characterised in that** the blowing-in of sterile air following the formation of the peroxide condensate film takes place in at least two separate steps each of which corresponds to one phased step in the transport of the bottles.

9. Procedure in accordance with one of the claims 1 to 8, **characterised in that** the sterile air is heated to an activation temperature of between 90 and 120 °C and, preferably, to about 110 °C.

10. Procedure in accordance with one of claims 1 to 9, **characterised in that** the sterile air that is blown in after the blowing-in of sterile air at activation temperature possesses a lower temperature.

11. Procedure in accordance with claim 10, **characterised in that** the lower temperature of the sterile air is between 75 and 85 °C and, preferably, about 80°C.

12. Procedure in accordance with one of claims 1 to 11, **characterised in that** the sterile air at activation temperature is blown into the inner space of the bottles at a flow speed of between 25 and 30 m / s, preferably about 28 m / s.

13. Procedure in accordance with one of the claims 1 to 12, **characterised in that** the sterile air at a lower temperature is blown into the inner space of the bottles at a flow speed of between 70 and 90 m / s and, preferably, about 80 m / s.

14. Procedure in accordance with one of claims 1 to 12, **characterised in that** on each occasion the sterile air is blown in for a period of between 1 and 3 seconds and, preferably, for about 2 seconds.

15. Procedure in accordance with one or more of claims 1 to 14, **characterised in that** about 0.15 ml of peroxide is introduced per 100 cm² of the surface of the inner space in the bottles.

## Revendications

1. Procédé pour la stérilisation de récipients avancés en cycle le long d'une voie de transport par utilisation d'un agent de stérilisation, dans lequel on insuffle un aérosol de peroxyde chauffé dans l'espace intérieur des récipients et il se forme un film de condensat de peroxyde sur la paroi intérieure des récipients et dans lequel on insuffle ensuite de l'air chauffé dans l'espace intérieur des récipients, jusqu'à ce que le condensat d'aérosol soit évaporé, **caractérisé en ce que**, pour la stérilisation de bouteilles en matériau synthétique sensible à la température, en particulier de bouteilles en PET, pour la formation du film de condensat, on n'insuffle l'aérosol de peroxyde dans les bouteilles qu'à une température de début de stérilisation, on insuffle ensuite jusqu'à la décomposition du film de condensat par évaporation de l'air stérile avec une température d'activation supérieure à la température de début de stérilisation de l'aérosol de peroxyde et on évacue ensuite les restes de peroxyde de l'espace intérieur des bouteilles par une insufflation d'air stérile effectuée dans une autre étape séparée.

2. Procédé selon la revendication 1, **caractérisé en ce que** le peroxyde est atomisé en aérosol à la température ambiante et est chauffé à la température de début de stérilisation sur le chemin vers l'espace intérieur des bouteilles.

3. Procédé selon les revendications 1 ou 2, **caractérisé en ce que** l'aérosol de peroxyde est chauffé avant l'introduction dans les bouteilles à environ 60 à 90°C, de préférence à environ 70 à 80°C, comme température de début de stérilisation.

4. Procédé selon l'une quelconque des revendications 1 à 3, **caractérisé en ce que** l'aérosol de peroxyde est introduit avant son insufflation dans les bouteilles dans un flux d'air stérile chauffé à la température d'activation et est chauffé par celui-ci sur le chemin vers l'espace intérieur des bouteilles à la température de début de stérilisation.

5. Procédé selon l'une quelconque des revendications 1 à 3, **caractérisé en ce que** l'aérosol de peroxyde et l'air stérile sont guidés séparément jusqu'à l'entrée dans l'espace intérieur des bouteilles.

6. Procédé selon l'une quelconque des revendications 1 à 5, **caractérisé en ce que** l'introduction de l'aérosol de peroxyde dans l'espace intérieur des bouteilles est réalisée en au moins deux étapes séparées consécutives.

7. Procédé selon l'une quelconque des revendications 1 à 6, **caractérisé en ce que** l'insufflation d'air chaud stérile chauffé à la température d'activation dans l'espace intérieur des bouteilles est précédée d'au moins une pause d'imprégnation suivant l'insufflation de l'aérosol de peroxyde, correspondant à au moins un cycle de transport des bouteilles.

8. Procédé selon l'une quelconque des revendications 1 à 7, **caractérisé en ce que** l'insufflation d'air stérile effectuée après la formation du film de condensat de peroxyde est réalisée en au moins deux étapes séparées, correspondant à chaque fois à un cycle de transport des bouteilles.

9. Procédé selon l'une quelconque des revendications 1 à 8, **caractérisé en ce que** l'air stérile est chauffé à une température d'activation d'environ 90 à 120°C, de préférence d'environ 110°C.

10. Procédé selon l'une quelconque des revendications 1 à 9, **caractérisé en ce** l'air stérile insufflé après l'insufflation d'air stérile à la température d'activation présente une température diminuée.

11. Procédé selon la revendication 10, **caractérisé en ce que** la température diminuée de l'air stérile est d'environ 75 à 85°C, de préférence d'environ 80°C.

12. Procédé selon l'une quelconque des revendications 1 à 11, **caractérisé en ce que** l'air stérile à la température d'activation est insufflé à une vitesse d'écoulement d'environ 25 à 30 m/s, de préférence d'environ 28 m/s, dans l'espace intérieur des bouteilles.

13. Procédé selon l'une quelconque des revendications 1 à 12, **caractérisé en ce que** l'air stérile à la température diminuée est insufflé à une vitesse d'écoulement d'environ 70 à 90 m/s, de préférence d'environ 80 m/s, dans l'espace intérieur des bouteilles.

14. Procédé selon l'une quelconque des revendications 1 à 12, **caractérisé en ce que** l'insufflation d'air stérile est réalisée à chaque fois pendant un laps de temps d'environ 1 à 3 secondes, de préférence d'environ 2 secondes.

15. Procédé selon l'une quelconque ou plusieurs des revendications 1 à 14, **caractérisé en ce qu'**on introduit dans les bouteilles environ 0,15 ml de peroxyde par 100 cm² de surface de l'espace intérieur des bouteilles.
